# EUROPEAN PATENT APPLICATION

(11) **EP 4 438 038 A1**
(43) Date of publication of application: **02.10.2024**
(21) Application number: 23165873.3
(22) Date of filing: 31.03.2023
(51) Int. Cl.: A61K 31/202, A61K 31/593, A61K 35/745, A61K 35/747, A61P 3/04, A61P 19/02

(54) **COMPOSITIONS FOR USE IN IMPROVING MOBILITY**

(71) Applicant: GlaxoSmithKline Consumer Healthcare (UK) IP Limited, Weybridge Surrey KT13 0NY (GB)
(72) Inventor: The designation of the inventor has not yet been filed
(74) Representative: Haleon Patent Department

(57) **Abstract**

The present invention relates to compositions for use in improving mobility and physical function in human subjects. Particularly, the compositions are for oral administration and comprise a probiotic component and an oil component. More particularly, the combination comprises (i) *Bifidobacterium animalis, Bifidobacterium lactis, Lactobacillus paracasei* and *Lactobacillus acidophilus;* (ii) one or more Omega-3 fatty acids; and (iii) Vitamin D.

## Description

### Field of the Invention

The present invention relates to compositions for use in improving mobility and physical function in human subjects. Particularly, the compositions are for oral administration and comprise a probiotic component and an oil component. More particularly, the combination comprises (i) *Bifidobacterium animalis, Bifidobacterium lactis, Lactobacillus paracasei* and *Lactobacillus acidophilus;* (ii) one or more Omega-3 fatty acids and (iii) Vitamin D.

### Background of the Invention

Mobility refers to the ability of an individual to move their body freely and easily, particularly without pain or discomfort. It may be assessed using standard performance-based tests or with patient reporting of their difficulty in carrying out specific tasks. Studies have shown that mobility is amongst the most frequently cited health problems of ageing individuals. Mobility issues include joint pain, weight gain and lack of joint flexibility, where joint pain and flexibility have the highest impact on quality of life. Whilst certain interventions are available, there remains a need for compositions such as supplements that provide mobility benefits. In particular, there remains a need for compositions that may be used to improve mobility and physical function in overweight and obese subjects with a body mass index (BMI) of at least 25kg/m², particularly adult subjects.

### Summary of the Invention

In a First Aspect, there is provided a combination of (i) *Bifidobacterium animalis, Bifidobacterium lactis, Lactobacillus paracasei* and *Lactobacillus acidophilus;* (ii) one or more Omega-3 fatty acids; and (iii) Vitamin D for use in improving mobility and physical function in a human subject by oral administration.

Particularly, the one or more Omega-3 fatty acids comprise eicosapentaenoic acid (EPA) and docosahexaenoic acid (DHA).

Particularly, the Vitamin D is vitamin D3 (cholecalciferol) and/or vitamin D2 (ergocalciferol), preferably vitamin D3 (cholecalciferol).

Particularly, the combination comprises at least 2.5×10⁹ colony forming units (CFU) of each of *Bifidobacterium animalis, Bifidobacterium lactis, Lactobacillus paracasei* and *Lactobacillus acidophilus.* In certain embodiments, the *Lactobacillus paracasei* is strain LCP-37 (deposited in the American Type Culture Collection as SD5275). In certain embodiments, the *Lactobacillus acidophilus* is strain NCFM^{®}. This strain may also be referred to as *Lactobacillus acidophilus* ATCC 700396, NCK56 and N2. In certain embodiments, the *Bifidobacterium animalis* is *Bifidobacterium animalis subsp. Lactis,* more particularly *Bifidobacterium animalis subsp. Lactis* strain Bi-07^{®} (also referred to as *Bifidobacterium lactis* Bi-07). In certain embodiments, the *Bifidobacterium lactis* is strain BI-04 (Also known as *Bifidobacterium animalis subsp lactis* BI-04^{®}). Each of these strains is widely available, both commercially and from culture collections.

In certain embodiments, the composition comprises around 640 mg of omega-3 fatty acids. Preferably, the combination comprises at least 300mg of EPA (eicosapentaenoic acid) and at least 220mg of DHA (docosahexaenoic acid). Preferably the one or more Omega-3 fatty acids are obtained from fish oil. In certain embodiments, the composition comprises around 1100 mg fish oils. In particular embodiments, the composition comprises about 1100mg fish oil comprising about 640 mg omega-3 of which about 300 mg is eicosapentaenoic acid (EPA) and about 220 mg is docosahexaenoic acid (DHA).

Particularly, the combination comprises at least 200 IU of Vitamin D.

In certain embodiments, the human subject has a body mass index (BMI) of at least 25 kg/m², particularly at least 28 kg/m². In certain embodiments, the human subject has a body mass index (BMI) of from 28 kg/m² to 29.9 kg/m². In certain embodiments, the human subject has a body mass index (BMI) of 30 kg/m² or more. In certain embodiments, the human subject has a body mass index (BMI) of from 30 kg/m² to 40 kg/m².

Particularly, the human subject is at least 25 years of age. More particularly, the human subject is from 25 to 65 years of age.

In preferred embodiments, the combination is a composition comprising 2.5×10⁹ colony forming units of *Bifidobacterium animalis,* 2.5×10⁹ colony forming units of *Bifidobacterium lactis,* 2.5×10⁹ colony forming units of *Lactobacillus paracasei,* 2.5×10⁹ colony forming units of *Lactobacillus acidophilus,* 300mg of EPA, 220mg of DHA and 200 IU of Vitamin D. More particularly, the combination is a composition comprising 2.5×10⁹ colony forming units of *Bifidobacterium animalis subsp. Lactis* strain Bi-07^{®}, 2.5×10⁹ colony forming units of *Bifidobacterium lactis* strain BI-04, 2.5×10⁹ colony forming units of *Lactobacillus paracasei* strain LCP-37, 2.5×10⁹ colony forming units of *Lactobacillus acidophilus* strain NCFM@, 300mg of EPA, 220mg of DHA and 200 IU of Vitamin D. Yet more particularly, the combination is a composition comprising 2.5×10⁹ colony forming units of *Bifidobacterium animalis subsp. Lactis* strain Bi-07^{®}, 2.5×10⁹ colony forming units of *Bifidobacterium lactis* strain BI-04, 2.5×10⁹ colony forming units of *Lactobacillus paracasei* strain LCP-37, 2.5×10⁹ colony forming units of *Lactobacillus acidophilus* strain NCFM@, 300mg of EPA, 220mg of DHA and 200 IU of Vitamin D3 (cholecalciferol).

In certain embodiments, the combination may be provided as a kit. Particularly a kit comprising (i) a probiotic composition comprising 2.5×10⁹ colony forming units of *Bifidobacterium animalis,* 2.5×10⁹ colony forming units of *Bifidobacterium lactis,* 2.5×10⁹ colony forming units of *Lactobacillus paracasei,* 2.5×10⁹ colony forming units of *Lactobacillus acidophilus* and (ii) a fish oil composition comprising 300mg of EPA, 220mg of DHA and 200 IU of Vitamin D. More particularly, a kit comprising (i) a probiotic composition comprising 2.5×10⁹ colony forming units of *Bifidobacterium animalis,* 2.5×10⁹ colony forming units of *Bifidobacterium lactis,* 2.5×10⁹ colony forming units of *Lactobacillus paracasei,* 2.5×10⁹ colony forming units of *Lactobacillus acidophilus* and (ii) a fish oil composition comprising 300mg of EPA, 220mg of DHA and 200 IU of Vitamin D. Yet more particularly, a kit comprising (i) a probiotic composition comprising 2.5×10⁹ colony forming units of *Bifidobacterium animalis subsp. Lactis* strain Bi-07^{®}, 2.5×10⁹ colony forming units of *Bifidobacterium lactis* strain BI-04, 2.5×10⁹ colony forming units of *Lactobacillus paracasei* strain LCP-37, 2.5×10⁹ colony forming units of *Lactobacillus acidophilus* strain NCFM^{®} and (ii) a fish oil composition comprising 300mg of EPA, 220mg of DHA and 200 IU of Vitamin D. Still yet more particularly, a kit comprising (i) a probiotic composition comprising 2.5×10⁹ colony forming units of *Bifidobacterium animalis subsp. Lactis* strain Bi-07^{®}, 2.5×10⁹ colony forming units of *Bifidobacterium lactis* strain BI-04, 2.5×10⁹ colony forming units of *Lactobacillus paracasei* strain LCP-37, 2.5×10⁹ colony forming units of *Lactobacillus acidophilus* strain NCFM^{®} and (ii) a fish oil composition comprising 300mg of EPA, 220mg of DHA and 200 IU of Vitamin D3 (cholecalciferol).

In some embodiments, the combination is formulated into a single dosage form suitable for oral administration, such as in the form of a capsule, gel, liquid dispersion, pill, powder or suspension.

In other embodiments, the combination is formulated into several dosage forms, particularly at least two dosage forms, such as two or three dosage forms. In certain embodiments, the *Bifidobacterium animalis, Bifidobacterium lactis, Lactobacillus paracasei* and *Lactobacillus acidophilus* are formulated into a first dosage form and the one or more Omega-3 fatty acids and Vitamin D are formulated into a second dosage form. Particularly the first and second dosage forms are administered concurrently or sequentially, for example, one after the other.

The combination is provided in dosage forms suitable for oral administration, such as in the form of capsules, gels, liquid dispersions, pills, powders or suspensions.

In some embodiments, the probiotic composition is provided in the form of a capsule, a gel, a liquid dispersion, a pill, a powder, or a suspension. Particularly, the probiotic composition is provided in the form of a capsule. In some embodiments, the fish-oil composition is provided in the form of a capsule, a gel, a liquid dispersion, a pill, a powder, or a suspension. Particularly, the fish-oil composition is provided in the form of a capsule. More particularly, the combination the *Bifidobacterium animalis, Bifidobacterium lactis, Lactobacillus paracasei* and *Lactobacillus acidophilus* are formulated into a capsule and the one or more Omega-3 fatty acids and Vitamin D are formulated into a capsule.

In certain embodiments, the probiotic composition is administered in two doses per day, each dose comprising 1.25×10⁹ colony forming units of *Bifidobacterium animalis,* 1.25×10⁹ colony forming units of *Bifidobacterium lactis,* 1.25×10⁹ colony forming units of *Lactobacillus paracasei* and 1.25×10⁹ colony forming units of *Lactobacillus acidophilus.* In certain embodiments, the fish-oil composition is administered in two doses per day, each dose comprising 150mg of EPA, 110mg of DHA and 100 IU of Vitamin D.

Particularly, the probiotic composition is administered in two capsules per day (each capsule comprising 1.25×10⁹ colony forming units of *Bifidobacterium animalis,* 1.25×10⁹ colony forming units of *Bifidobacterium lactis,* 1.25×10⁹ colony forming units of *Lactobacillus paracasei* and 1.25×10⁹ colony forming units of *Lactobacillus acidophilus*) and the fish-oil composition is also administered in two capsules per day (each capsule) comprising 150mg of EPA, 110mg of DHA and 100 IU of Vitamin D. Thus, the human subject would be taking four capsules per day by oral administration providing 2.5×10⁹ colony forming units of *Bifidobacterium animalis,* 2.5×10⁹ colony forming units of *Bifidobacterium lactis,* 2.5×10⁹ colony forming units of *Lactobacillus paracasei,* 2.5×10⁹ colony forming units of *Lactobacillus acidophilus,* 300mg of EPA, 220mg of DHA and 200 IU of Vitamin D.

In a Second Aspect of the Invention, there is provided a method of maintaining or improving mobility and physical function in a human subject, wherein said method comprises administering to the subject a combination of (i) *Bifidobacterium animalis, Bifidobacterium lactis, Lactobacillus paracasei* and *Lactobacillus acidophilus;* (ii) one or more Omega-3 fatty acids; and (iii) Vitamin D.

There is also provided a method of maintaining or improving mobility and physical function in a human subject, wherein said method comprises administering to the human subject a combination comprising 2.5×10⁹ colony forming units of *Bifidobacterium animalis,* 2.5×10⁹ colony forming units of *Bifidobacterium lactis,* 2.5×10⁹ colony forming units of *Lactobacillus paracasei,* 2.5×10⁹ colony forming units of *Lactobacillus acidophilus,* 300mg of EPA, 220mg of DHA and 200 IU of Vitamin D.

There is also provided a method of maintaining or improving mobility and physical function in a human subject, wherein said method comprises administering to the human subject a combination comprising 2.5×10⁹ colony forming units of *Bifidobacterium animalis subsp. Lactis* strain Bi-07^{®}, 2.5×10⁹ colony forming units of *Bifidobacterium lactis* strain BI-04, 2.5×10⁹ colony forming units of *Lactobacillus paracasei* strain LCP-37, 2.5×10⁹ colony forming units of *Lactobacillus acidophilus* strain NCFM@, 300mg of EPA, 220mg of DHA and 200 IU of Vitamin D.

### Brief description of Figures

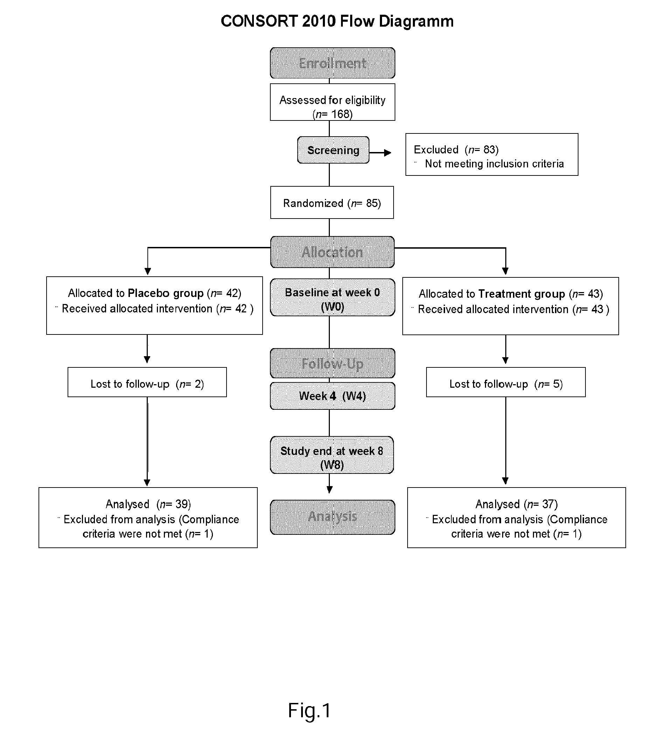

- **Figure 1.**: A study designed as a randomized, double-blind, placebo-controlled parallel-group study is shown as a flow diagram. A total of 85 participants were randomized into two groups at baseline (W0). 42 participants were in the placebo group and 43 participants in the treatment group. The intervention took 8 weeks (W8) and the same parameters were measured at W0 and W8. Abbreviations: W, week.

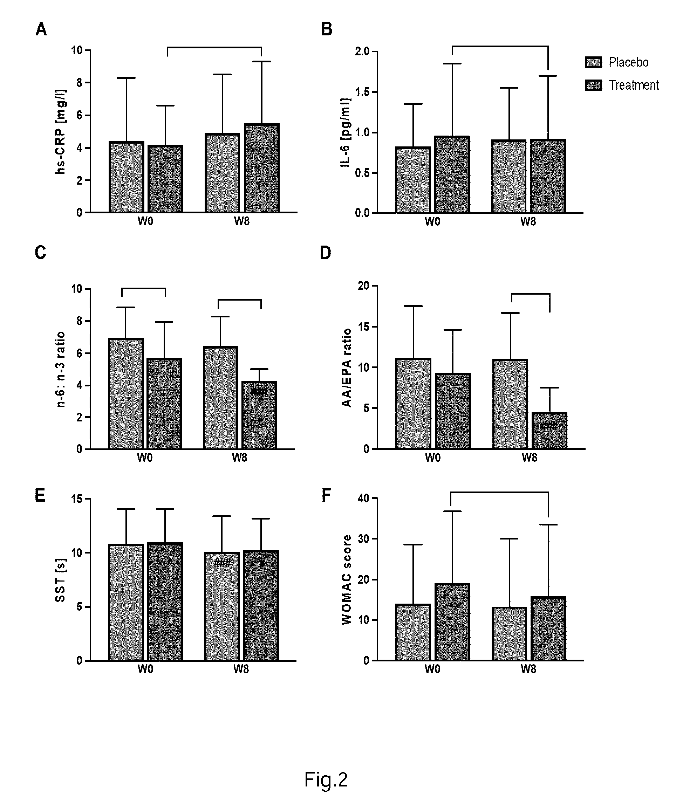

- **Figure 2.**: Effect of Supplementation on hs-CRP Levels (A) and IL-6 levels (B) comparing the placebo and treatment group at study start (W0) and study end (W8). Further FA plasma ratios were calculated as the n-6:n-3 ratio (C) and the AA/EPA ratio (D), as well difference within and between groups at physical function tests SST (E), and WOMAC score (F) before (W0) and after (W8) the eight-week intervention. Values are expressed as mean ± SD from 39 (placebo) and 37 (treatment) participants. Abbreviations: W, week; hs-CRP, high-sensitivity c-reactive protein, IL, interleukin; n, omega; AA, arachidonic acid; SST, Sit- and Stand test; WOMAC, Western Ontario and McMaster Osteoarthritis Index. Statistics: # indicates differences between W0 and W8 within the treatment and placebo groups (calculated by either paired t-test or Wilcoxon test). * Indicates differences between placebo and treatment group at W0 or W8. #,*p < 0.05; ##,**p < 0.01; and ###,***p < 0.001 (D-L, calculated by either unpaired t-test or Mann-Whitney U test).

### Description of the Invention

The present Inventors have discovered that daily supplementation with a combination of probiotics (*Bifidobacterium animalis, Bifidobacterium lactis, Lactobacillus paracasei* and *Lactobacillus acidophilus*) Omega-3 fatty acids and Vitamin D elicited improvements in mobility and physical function in human subjects, evaluated using the Western Ontario and McMaster Universities Osteoarthritis Index (WOMAC). WOMAC is a widely used measure of symptoms and physical disability, originally developed for people with osteoarthritis of the hip and/or knee (McConnell et al. 2001, Arthritis Care & Research 45:453-461).

Thus, (ii) one or more Omega-3 fatty acids; and (iii) Vitamin D for use in improving mobility and physical function in a human subject by oral administration. Particularly the improvement in mobility and physical function is measured by a WOMAC score.

### Probiotic component

The term "probiotic" refers to living bacteria that are administered to a subject in an appropriate amount for the purpose of obtaining a health-promoting effect. Probiotic bacteria can survive in the environment of the gastrointestinal tract after ingestion and potentially have the ability to form colonies in the intestines of the subject.

The present Invention is based on the use of a combination of at least four different strains of probiotic bacteria, specifically: *Bifidobacterium animalis, Bifidobacterium lactis, Lactobacillus paracasei* and *Lactobacillus acidophilus.* have identified four *Bifidobacterium animalis, Bifidobacterium lactis, Lactobacillus paracasei* and *Lactobacillus acidophilus.* Particularly, the *Bifidobacterium animalis* is a *Bifidobacterium animalis subsp. Lactis* strain, more particularly *Bifidobacterium animalis subsp. Lactis* strain Bi-07^{®}. Bifidobacterium lactis Bi-07^{®} is a gram-positive, anaerobic, rod-shaped bacterium generally found in the large intestines of most mammals. This strain is widely available from commercial sources and was recently reclassified, being formerly known as Bifidobacterium lactis Bi-07 or Bifidobacterium lactis ATCC SD5220.

*Lactobacillus paracasei* is a Gram-positive, non-spore forming, homofermentative rod that is a common inhabitant of the human intestinal tract and also found naturally in fermented vegetables, milk, and meat. Particularly, the *Lactobacillus paracasei* is *Lactobacillus paracasei* strain LCP-37 (deposited in the American Type Culture Collection as SD5275). The strain may also be referred to as *Lacticaseibacillus paracasei* Lpc-37^{®}.

*Lactobacillus acidophilus* is a Gram-positive, non-spore forming bacterium found in the human intestinal tract, mouth and certain fermented dairy products. Particularly, the *Lactobacillus acidophilus* is *Lactobacillus acidophilus* strain NCFM^{®}.

This strain may also be referred to as *Lactobacillus acidophilus* ATCC 700396, NCK56 and N2.

*Bifidobacterium lactis* is an anaerobic, gram-positive, rod-shaped bacterium which can be found in the large intestines of many mammals, including humans. Particularly, the *Bifidobacterium lactis* is *Bifidobacterium lactis* strain BI-04 (Also known as also known as DGCC2908 and RB 4825, available in the American Type Culture Collection as SD5219).

Strains of *Bifidobacterium animalis, Bifidobacterium lactis, Lactobacillus paracasei* and *Lactobacillus acidophilus* and each of the specific strains referred to above (Bi-07^{®}, LCP-37^{®}, NCFM^{®} and BI-04^{®}) is widely available, both commercially and from culture collections.

The term "colony-forming units" (CFUs) refers to an estimate of a number of viable bacterial cells in the combination. Viable cells are able to multiply under controlled conditions. For solid dosage forms, CFUs may be provided as CFU/g or CFU/mL for liquids. Particularly, the combination for use comprises from about 5×10⁹ colony forming units to about 10×10⁹ of colony forming units of bacteria.

Particularly, the ratio of CFUs of *Bifidobacterium animalis:Bifidobacterium lactis: Lactobacillus paracasei: Lactobacillus acidophilus* is about 1:1:1:1. Particularly, the combination for use comprises at least 1.25×10⁹ colony forming units of each strain of bacteria, at least 2.5×10⁹ colony forming units of each strain of bacteria. For example, from about 1.25×10⁹ to about 2.5×10⁹ colony forming units of each strain of bacterium. In certain embodiments, the combination for use comprises 1.25×10⁹ colony forming units of *Bifidobacterium animalis,* 1.25×10⁹ colony forming units of *Bifidobacterium lactis,* 1.25×10⁹ colony forming units of *Lactobacillus paracasei* and 1.25×10⁹ colony forming units of *Lactobacillus acidophilus.* In other embodiments, the combination for use comprises 2.5×10⁹ colony forming units of *Bifidobacterium animalis,* 2.5×10⁹ colony forming units of *Bifidobacterium lactis,* 2.5×10⁹ colony forming units of *Lactobacillus paracasei* and 2.5×10⁹ colony forming units of *Lactobacillus acidophilus.*

### Omega-3 fatty acids

Omega-3 fatty acids, also referred to in the art as n-3 fatty acids, are polyunsaturated fatty acids (PUFAs) found widely in nature. Examples include, hexadecatrienoic acid (HTA); a-Linolenic acid (ALA); Stearidonic acid (SDA); Eicosatrienoic acid (ETE); Eicosatetraenoic acid (ETA); Eicosapentaenoic acid (EPA); Heneicosapentaenoic acid (HPA); Docosapentaenoic acid (DPA); Clupanodonic acid; Docosahexaenoic acid (DHA); Tetracosapentaenoic acid; and Tetracosahexaenoic acid (Nisinic acid).

The three types of omega-3 fatty acids important for human physiology are α-linolenic acid (ALA), eicosapentaenoic acid (EPA) and docosahexaenoic acid (DHA). DHA and EPA are found in algae and fish whilst ALA is generally found in plants.

As used herein, the term "omega-3 fatty acids" includes both natural and synthetic omega-3 fatty acids, as well as pharmaceutically acceptable esters, free acids, triglycerides, derivatives, conjugates, precursors, salts, and mixtures thereof. Omega-3 fatty acids

Omega-3 fatty acids for use in the combinations described herein can have a high content of eicosapentaenoic acid (EPA) as well as docosahexaenoic acid (DHA). Preferred omega-3 fatty acids are EPA or DHA, triglycerides thereof, ethyl esters thereof and mixtures thereof. Particularly, the omega-3 fatty acids comprise EPA, DHA, or a combination thereof. More particularly, the omega-3 fatty acids comprise EPA and DHA.

The one or more omega-3 fatty acid (or their esters, derivatives, precursors, salts and mixtures thereof) can be used either in pure form or as a component of an oil. The one or more omega-3 fatty acid may be derived from animal oils and/or non-animal oils. For example, the one or more omega-3 fatty acid is derived from at least one oil chosen from marine oil, algae oil, plant-based oil, and microbial oil. Marine oils include, for example, fish oil, such as tuna fish oil, krill oil, and lipid composition derived from fish. Plant-based oils include, for example, flaxseed oil, canola oil, mustard seed oil, and soybean oil. Microbial oils include, for example, products by Martek.

Particularly, the one or more omega-3 fatty acid is derived from a marine oil, such as a fish oil, more particularly a purified fish oil. As used herein, the term "fish oil" refers to an oil derived from a fish or fish tissue. Fish oil is available commercially. Fish oil comprises omega-3 fatty acids including DHA and EPA. The fish oil may be derived from one or more cold-water fish species, which are known for having a high omega-3 fatty acid content. Suitable fish oil sources include deep-sea fish, shark, salmon, cod, salmon, bonito, mackerel, Atlantic mackerel, haddock, herring, mahi mahi, menhaden, mackerel, caplin, tilapia, pacific saury, krill, anchovies, pollock, trout, whitefish, tuna, smelt, shad, and sardines, cold-water fish as described elsewhere herein, and the like.

Preferably, the fish oil has a high omega-3 fatty acid oil content, such as 50% or higher, more preferably, 70% or higher, most preferably 80% or higher.

The one or more omega-3 fatty acid and/or fish oil comprising the one or more omega-3 fatty acids can be highly refined, for example highly enriched beyond the initial content of omega-3 fatty acids. In some embodiments they can comprise a minimum of 95% weight, e.g., 96% weight, 97% weight, 98% weight or greater, of monomeric triglycerides. In some embodiments of any of the aspects, these compositions can comprise less than 1% weight percent of oxidized triglycerides, less than 0.2% weight percent of trimeric and oligomeric triglycerides.

In some embodiments, the total content of EPA and DHA in the fish oil is from about 25%-70% weight. In some embodiments, the total content of EPA and DHA in the fish oil is from about 35%-70% weight, such as 35%-60%, such as 40%-60%, such as 40%-50%.

Particularly, the composition for use comprises at least 320 mg of omega-3 fatty acids, at least 640 mg of omega-3 fatty acids, such as from about 320 mg to about 640 mg of omega-3 fatty acids. Particularly, the at least one omega-3 fatty acid comprises at least 100mg of EPA, at least 150 mg of EPA or at least 300mg of EPA. Particularly, the at least one omega-3 fatty acid comprises from about 100mg of EPA to about 300mg of EPA, more particularly from about 150 mg of EPA or about 300mg of EPA, yet more particularly 150mg of EPA or 300mg EPA. Particularly, the at least one omega-3 fatty acid comprises at least 100mg of DHA, at least 110 mg of DHA or at least 220mg of DHA. Particularly, the at least one omega-3 fatty acid comprises from about 100mg of DHA to about 220mg of DHA, more particularly from about 110 mg of DHA or about 300mg of DHA, yet more particularly 110mg of DHA or 220mg DHA. Particularly, the combination comprises at least 300mg of EPA and at least 220mg of DHA. Particularly, the combination comprises about 300mg of EPA and about 220mg of DHA.

Preferably the one or more Omega-3 fatty acids are obtained from fish oil. In certain embodiments, the composition comprises at least 550mg of fish oil, at least 1100mg of fish oil, such as from about 550mg to about 1100 mg of fish oil.

Preferably, the composition for use comprises about 1100mg fish oil comprising about 640 mg omega-3 of which about 300 mg is eicosapentaenoic acid (EPA) and about 220 mg is docosahexaenoic acid (DHA).

Whilst fish oils may have a cholesterol content of from about 4000 to about 12000 ppm, the cholesterol content of the fish oil and/or omega-3 fatty acids as used herein (e.g., after refinement or enrichment) may be lower, for example, comprising less than 2500 ppm or less than 1500 ppm.

### Vitamin D

The term "vitamin D" encompasses a group of fat- soluble prohormones, as well as metabolites and analogues. The main forms of vitamin D are vitamin D2 (ergocalciferol) and vitamin D3 (cholecalciferol). Vitamin D3 is the endogenous form of vitamin D, which forms in the skin under the influence of sunlight. Generally, Vitamin D2 is used an exogenous form of vitamin D to fortify food. Unless otherwise indicated, the term Vitamin D refers to any form or forms of Vitamin D, including Vitamin D metabolites such as 25-hydroxy- Vitamin D or 1,25 dihydroxy Vitamin D. Preferably, the Vitamin D is vitamin D3 (cholecalciferol) and/or vitamin D2 (ergocalciferol). More preferably the Vitamin D is vitamin D3 (cholecalciferol).

Particularly, the combination for use comprises at least 100 IU (International Units) of Vitamin D or at least 200 IU of Vitamin D, such as from about 100 IU to about 200 IU of Vitamin D, particularly 100 IU or 200 IU of Vitamin D. More particularly, the combination for use comprises at least 100 IU (International Units) of Vitamin D3 or at least 200 IU of Vitamin D3, such as from about 100 IU to about 200 IU of Vitamin D3, particularly 100 IU or 200 IU of Vitamin D3.

### Human Subject

The human subject may have a body mass index (BMI) of at least 25 kg/m², particularly at least 28 kg/m². In certain embodiments, the human subject has a body mass index (BMI) of from 28 kg/m² to 29.9 kg/m². In certain embodiments, the human subject has a body mass index (BMI) of 30 kg/m² or more. In certain embodiments, the human subject has a body mass index (BMI) of from 30 kg/m² to 40 kg/m². The human subject may be overweight (BMI 25-29.9 kg/m²) and/or obese (BMI ≥30 kg/m²)

Particularly, the human subject is an adult, more particularly the human subject is an adult at least 25 years of age. More particularly, the human subject is an adult of from 25 to 65 years of age. In certain embodiments, the human subject is an adult of from 25 to 50 years of age. In certain embodiments, the human subject is an adult of from 51 to 65 years of age. In some embodiments, the human subject is male. In other embodiments, the human subject is female.

### Dosage Form

Combinations for use are suitable for oral administration and may be formulated into any dosage form that may be administered through the oral cavity and into the gastrointestinal tract of a human subject. In some embodiments, the combination is formulated into a single dosage form suitable for oral administration, such as in the form of a capsule, gel, liquid dispersion, pill, powder or suspension.

In other embodiments, the combination is formulated into several dosage forms, particularly at least two dosage forms, such as two or three dosage forms.

In certain embodiments, the *Bifidobacterium animalis, Bifidobacterium lactis, Lactobacillus paracasei* and *Lactobacillus acidophilus* are formulated into a first dosage form and the one or more Omega-3 fatty acids and Vitamin D are formulated into a second dosage form. Particularly the first and second dosage forms are administered concurrently or sequentially, for example, one after the other.

The combination is provided in dosage forms suitable for oral administration, such as in the form of capsules, gels, liquid dispersions, pills, powders or suspensions. In some embodiments, the probiotic composition is provided in the form of a capsule, a gel, a liquid dispersion, a pill, a powder, or a suspension. Particularly, the probiotic composition is provided in the form of a capsule. In some embodiments, the fish-oil composition is provided in the form of a capsule, a gel, a liquid dispersion, a pill, a powder, or a suspension. Particularly, the fish-oil composition is provided in the form of a capsule.

Particularly, the probiotic component of the combination for use (comprising the *Bifidobacterium animalis, Bifidobacterium lactis, Lactobacillus paracasei* and *Lactobacillus acidophilus*) is formulated into a first capsule and the one or more Omega-3 fatty acids and Vitamin D are formulated into a second capsule.

In certain embodiments, the probiotic composition is administered in two doses per day, each dose comprising 1.25×10⁹ colony forming units of *Bifidobacterium animalis,* 1.25×10⁹ colony forming units of *Bifidobacterium lactis,* 1.25×10⁹ colony forming units of *Lactobacillus paracasei* and 1.25×10⁹ colony forming units of *Lactobacillus acidophilus.* Thus, a human subject would receive 2.5×10⁹ CFUs of each of the four strains of bacteria per day, a total of 10×10⁹ CFUs of bacteria per day.

In certain embodiments, the fish-oil composition is administered in two doses per day, each dose comprising 150mg of EPA, 110mg of DHA and 100 IU of Vitamin D.

Particularly, the probiotic composition is administered in two capsules per day (each capsule comprising 1.25×10⁹ colony forming units of *Bifidobacterium animalis,* 1.25×10⁹ colony forming units of *Bifidobacterium lactis,* 1.25×10⁹ colony forming units of *Lactobacillus paracasei* and 1.25×10⁹ colony forming units of *Lactobacillus acidophilus*) and the fish-oil composition is also administered in two capsules per day, each capsule comprising 150mg of EPA, 110mg of DHA and 100 IU of Vitamin D. Thus, the human subject would be taking four capsules per day by oral administration providing 2.5×10⁹ colony forming units of *Bifidobacterium animalis,* 2.5×10⁹ colony forming units of *Bifidobacterium lactis,* 2.5×10⁹ colony forming units of *Lactobacillus paracasei,* 2.5×10⁹ colony forming units of *Lactobacillus acidophilus,* 300mg of EPA, 220mg of DHA and 200 IU of Vitamin D.

In certain embodiments, the combination for use comprises 2.5×10⁹ colony forming units of *Bifidobacterium animalis,* 2.5×10⁹ colony forming units of *Bifidobacterium lactis,* 2.5×10⁹ colony forming units of *Lactobacillus paracasei,* 2.5×10⁹ colony forming units of *Lactobacillus acidophilus,* 300mg of EPA, 220mg of DHA and 200 IU of Vitamin D. More particularly, the combination for use comprises 2.5×10⁹ colony forming units of *Bifidobacterium animalis subsp. Lactis* strain Bi-07^{®}, 2.5×10⁹ colony forming units of *Bifidobacterium lactis* strain BI-04, 2.5×10⁹ colony forming units of *Lactobacillus paracasei* strain LCP-37, 2.5×10⁹ colony forming units of *Lactobacillus acidophilus* strain NCFM@, 300mg of EPA, 220mg of DHA and 200 IU of Vitamin D. Yet more particularly, the combination for use comprises 2.5×10⁹ colony forming units of *Bifidobacterium animalis subsp. Lactis* strain Bi-07^{®}, 2.5×10⁹ colony forming units of *Bifidobacterium lactis* strain BI-04, 2.5×10⁹ colony forming units of *Lactobacillus paracasei* strain LCP-37, 2.5×10⁹ colony forming units of *Lactobacillus acidophilus* strain NCFM@, 300mg of EPA, 220mg of DHA and 200 IU of Vitamin D3 (cholecalciferol).

The combination for use may be provided as a kit, particularly a kit comprising (i) a probiotic composition and (ii) a fish oil composition. The kit may be packed in a bottle container or the like. For example, the probiotic composition may be packed into a first bottle container or the like and the fish oil composition may be packed into a second bottle container or the like.

The bottle container or the like may be a multi-dose package tailored for use over a certain period of time by the human subject. Multi-dose package can contain unit dosage forms in the appropriate number, in the appropriate dosage(s), and in the appropriate packaging (e.g., daily, monthly packaging, weekly packaging, etc.) for the claimed use. The number of unit dosages may vary depending number of days the bottle container or the like is intended to be used, and will vary depending on the number of unit dosages to be administered per day. For example, a multi-dose bottle container or the like can be a monthly package that includes daily discrete doses wherein the total number of daily doses present in the monthly package may be equal to the total number of days in the month, or a multiple thereof, in implementations in which more than one dose is being administered. For example, a monthly package may include from 30 to 210 doses, such as from 60 to 180 doses, or 90 to 120 doses, for example, in the form of capsules and the like.

In certain embodiments, the number of daily doses of probiotic composition is two and the number of daily doses of fish oil composition is two. In this case, a monthly pack would contain around 60 capsules of probiotic and around 60 capsules of fish oil composition.

Particularly the kit comprises (i) at least one probiotic composition comprising 2.5×10⁹ colony forming units of *Bifidobacterium animalis,* 2.5×10⁹ colony forming units of *Bifidobacterium lactis,* 2.5×10⁹ colony forming units of *Lactobacillus paracasei,* 2.5×10⁹ colony forming units of *Lactobacillus acidophilus* and (ii) at least one fish oil composition comprising 300mg of EPA, 220mg of DHA and 200 IU of Vitamin D. More particularly, a kit comprising (i) at least ones probiotic composition comprising 2.5×10⁹ colony forming units of *Bifidobacterium animalis,* 2.5×10⁹ colony forming units of *Bifidobacterium lactis,* 2.5×10⁹ colony forming units of *Lactobacillus paracasei,* 2.5×10⁹ colony forming units of *Lactobacillus acidophilus* and (ii) at least one fish oil composition comprising 300mg of EPA, 220mg of DHA and 200 IU of Vitamin D. Yet more particularly, a kit comprising (i) at least one probiotic composition comprising 2.5×10⁹ colony forming units of *Bifidobacterium animalis subsp. Lactis* strain Bi-07^{®}, 2.5×10⁹ colony forming units of *Bifidobacterium lactis* strain BI-04, 2.5×10⁹ colony forming units of *Lactobacillus paracasei* strain LCP-37, 2.5×10⁹ colony forming units of *Lactobacillus acidophilus* strain NCFM^{®} and (ii) at least one fish oil composition comprising 300mg of EPA, 220mg of DHA and 200 IU of Vitamin D. Still yet more particularly, a kit comprising (i) at least one probiotic composition comprising 2.5×10⁹ colony forming units of *Bifidobacterium animalis subsp. Lactis* strain Bi-07^{®}, 2.5×10⁹ colony forming units of *Bifidobacterium lactis* strain BI-04, 2.5×10⁹ colony forming units of *Lactobacillus paracasei* strain LCP-37, 2.5×10⁹ colony forming units of *Lactobacillus acidophilus* strain NCFM^{®} and (ii) at least one fish oil composition comprising 300mg of EPA, 220mg of DHA and 200 IU of Vitamin D3 (cholecalciferol).

### Improving mobility and physical function

The term "mobility and physical function" refers to the ability of an individual to move their body freely and easily. It can generally be assessed using standardised performance-based tests or with reports of difficulty in carrying out specific physical activities such as walking or climbing stairs. In the context of the present Invention, improvement in mobility and physical function may be assessed using the WOMAC index. Higher scores on the WOMAC indicate worse pain, stiffness, and functional limitations indicating lower mobility and physical function.

WOMAC test questions are typically scored on a scale of 0 to 4, corresponding to: None (0), Mild (1), Moderate (2), Severe (3), and Extreme (4). There are three categories (pain, stiffness and physical function) for each of which there are a number of assessment questions (five questions for pain, two questions for stiffness and seventeen questions for physical function). For example, the five questions relating to 'pain' are designed to evaluate pain experienced during five different situations: walking on a flat surface, going up or down stairs, sitting or lying in bed at night and standing upright. The patient's response to each question produces a score that is then summed to provide an aggregate score for each category, producing three subscale scores (pain, stiffness and physical function). The scores for each subscale are added together (with a possible score range of 0-20 for Pain, 0-8 for Stiffness, and 0-68 for Physical Function) to provide a total score (WOMAC index). Typically, improvement of mobility and physical function is assessed by a change in score (reduction) from a W0 baseline score (measured prior to start of treatment or first administration of the combination) to a W8 score measured eight weeks after the start of treatment. Particularly, the WOMAC questionnaire is completed by the human subject at week 0 (W0) and week 8 (W8).

In some embodiments the combination for use improves mobility and physical function as measured by WOMAC Pain subscale. In some embodiments the combination for use improves mobility and physical function as measured by WOMAC stiffness subscale. In some embodiments the combination for use improves mobility and physical function as measured by WOMAC physical function subscale. In some embodiments the combination for use improves mobility and physical function as measured by WOMAC index (total score).

Particularly, improvement in mobility and physical function is evidenced by a lower WOMAC Pain score, lower WOMAC stiffness score and/or lower WOMAC Physical Function score when compared to a baseline value prior to administration of the combination or at start of administration of the combination. Thus, more particularly, improvement in mobility and physical function is evidenced by a lower WOMAC Index score when compared to a baseline value prior to administration of the combination or at start of administration of the combination.

In certain embodiments, use of the combination improves mobility and physical function from at least four weeks after first administration of the combination or from at least eight weeks after first administration of the combination.

In some embodiments, the human subject achieves a statistically significant improvement in WOMAC index after administration of the combination for at least 8 weeks. In some embodiments, as a result of administration of the combination, the human subject achieves a lower WOMAC index score after 8 weeks of treatment than the subject had before treatment.

### Methods

The Invention also provides a method of maintaining or improving mobility and physical function in a human subject, wherein said method comprises administering to the subject a combination of (i) *Bifidobacterium animalis, Bifidobacterium lactis, Lactobacillus paracasei* and *Lactobacillus acidophilus;* (ii) one or more Omega-3 fatty acids; and (iii) Vitamin D.

There is also provided a method of maintaining or improving mobility and physical function in a human subject, wherein said method comprises administering to the human subject a combination comprising 2.5×10⁹ colony forming units of *Bifidobacterium animalis,* 2.5×10⁹ colony forming units of *Bifidobacterium lactis,* 2.5×10⁹ colony forming units of *Lactobacillus paracasei,* 2.5×10⁹ colony forming units of *Lactobacillus acidophilus,* 300mg of EPA, 220mg of DHA and 200 IU of Vitamin D.

There is also provided a method of maintaining or improving mobility and physical function in a human subject, wherein said method comprises administering to the human subject a combination comprising 2.5×10⁹ colony forming units of *Bifidobacterium animalis subsp. Lactis* strain Bi-07^{®}, 2.5×10⁹ colony forming units of *Bifidobacterium lactis* strain BI-04, 2.5×10⁹ colony forming units of *Lactobacillus paracasei* strain LCP-37, 2.5×10⁹ colony forming units of *Lactobacillus acidophilus* strain NCFM@, 300mg of EPA, 220mg of DHA and 200 IU of Vitamin D.

There is also provided a method of maintaining or improving mobility and physical function in a human subject, wherein said method comprises administering to the human subject a combination comprising 2.5×10⁹ colony forming units of *Bifidobacterium animalis subsp. Lactis* strain Bi-07^{®}, 2.5×10⁹ colony forming units of *Bifidobacterium lactis* strain BI-04, 2.5×10⁹ colony forming units of *Lactobacillus paracasei* strain LCP-37, 2.5×10⁹ colony forming units of *Lactobacillus acidophilus* strain NCFM@, 300mg of EPA, 220mg of DHA and 200 IU of Vitamin D3 (cholecalciferol).

In certain embodiments, the method comprises administering the combination as a first probiotic composition and a second fish oil composition wherein the first probiotic composition is administered in two doses per day, each dose comprising 1.25×10⁹ colony forming units of *Bifidobacterium animalis,* 1.25×10⁹ colony forming units of *Bifidobacterium lactis,* 1.25×10⁹ colony forming units of *Lactobacillus paracasei* and 1.25×10⁹ colony forming units of *Lactobacillus acidophilus* and wherein the second fish-oil composition is administered in two doses per day, each dose comprising 150mg of EPA, 110mg of DHA and 100 IU of Vitamin D.

Particularly, the probiotic composition is administered in two capsules per day (each capsule comprising 1.25×10⁹ colony forming units of *Bifidobacterium animalis,* 1.25×10⁹ colony forming units of *Bifidobacterium lactis,* 1.25×10⁹ colony forming units of *Lactobacillus paracasei* and 1.25×10⁹ colony forming units of *Lactobacillus acidophilus*) and the fish-oil composition is also administered in two capsules per day (each capsule) comprising 150mg of EPA, 110mg of DHA and 100 IU of Vitamin D. Thus, the human subject would be taking four capsules per day by oral administration providing 2.5×10⁹ colony forming units of *Bifidobacterium animalis,* 2.5×10⁹ colony forming units of *Bifidobacterium lactis,* 2.5×10⁹ colony forming units of *Lactobacillus paracasei,* 2.5×10⁹ colony forming units of *Lactobacillus acidophilus,* 300mg of EPA, 220mg of DHA and 200 IU of Vitamin D.

As discussed supra, improvement in mobility and physical function is evidenced by a lower WOMAC Pain score, lower WOMAC stiffness score and/or lower WOMAC Physical Function score when compared to a baseline value prior to administration of the combination or at start of administration of the combination. More particularly, improvement in mobility and physical function is evidenced by a lower WOMAC Index score when compared to a baseline value prior to administration of the combination or at start of administration of the combination.

In certain embodiments, the human subject has a body mass index (BMI) of at least 25 kg/m², particularly at least 28 kg/m². In certain embodiments, the human subject has a body mass index (BMI) of from 28 kg/m² to 29.9 kg/m². In certain embodiments, the human subject has a body mass index (BMI) of 30 kg/m² or more. In certain embodiments, the human subject has a body mass index (BMI) of from 30 kg/m² to 40 kg/m².

Particularly, the human subject is at least 25 years of age. More particularly, the human subject is from 25 to 65 years of age.

### GENERAL

The term "comprising" encompasses "including" e.g. a composition "comprising" X may include something additional e.g. X + Y. In some implementations, the term "comprising" refers to the inclusion of the indicated active agent, such as recited polypeptides, as well as inclusion of other active agents, and pharmaceutically acceptable carriers, excipients, emollients, stabilizers, etc., as are known in the pharmaceutical industry. In some implementations, the term "consisting essentially of" refers to a composition, whose only active ingredient is the indicated active ingredient(s), for example ibuprofen-arginine salt, however, other compounds may be included which are for stabilizing, preserving, etc. the formulation, but are not involved directly in the therapeutic effect of the indicated active ingredient. Use of the transitional phrase "consisting essentially" means that the scope of a claim is to be interpreted to encompass the specified materials or steps recited in the claim, and those that do not materially affect the basic and novel characteristic(s) of the claimed invention. See, In re Herz, 537 F.2d 549, 551-52, 190 USPQ 461, 463 (CCPA 1976) (emphasis in the original); see also MPEP § 2111.03. Thus, the term "consisting essentially of" when used in a claim of this invention is not intended to be interpreted to be equivalent to "comprising". The term "consisting of" and variations thereof means including and limited to (for example, the specific recited constituents or steps). In certain territories, the term "comprising an active ingredient consisting of" may be used in place of "consisting essentially". The term "about" in relation to a numerical value x is optional and means, for example, that the numerical value may comprise some variance around the stated number to allow for routine experimental fluctuation, measurement variance or to encompass minor deviations that may achieve substantially the same results as the stated number, such as x±10%, x±5%, x±4%, x±3%, x±2% or x±1%. The word "substantially" does not exclude "completely" e.g. a composition which is "substantially free" from Y may be completely free from Y. Where necessary, the word "substantially" may be omitted from the definition of the invention. Where methods refer to process steps, for example as (a), (b), (c), etc., these are intended to be sequential, i.e., step (c) follows step (b) which is preceded by step (a).

All references or patent applications cited within this patent specification are incorporated by reference herein. In order that this invention may be better understood, the following examples are set forth. These examples are for purposes of illustration only and are not to be construed as limiting the scope of the invention in any manner.

### Examples

### Example 1:

A study was designed to investigate the effect of eight-weeks of daily supplementation with a multi-strain probiotic (*Bifidobacterium animalis, Bifidobacterium lactis, Lactobacillus paracasei* and *Lactobacillus acidophilus*), n-3 PUFAs and vitamin D in overweight and obese subjects.

The study was designed as a randomized, double-blind, 1:1 placebo-controlled parallel-group study with four visits (Figure 1). Recruitment took place between July 2019 and September 2020. After randomization and during the study, there were a total of seven dropouts due to antibiotic treatment, personal reasons, or loss of follow-up without any reason given. In addition, two participants did not meet the compliance criteria and were thus excluded from the result analyses; 39 subjects from the placebo group and 37 subjects from the treatment group were finally analysed (Figure 1).

Overweight female and male subjects (body mass index (BMI) 28 - 29.9 kg/m²) and female and male subjects with obesity (BMI ≥ 30 - 40 kg/m²), aged between 25 and 65 years were screened for low-grade inflammation (serum hs-CRP level between 2 - 10 mg/L). Participants had to be free from chronic illness (except for obesity-associated diseases like hypertension, non-alcoholic fatty liver disease, and depression) and willing to adhere to certain dietary rules (i.e. no significant changes in diet, fibre intake, fluid intake throughout the study period, and no intake of probiotic products, n-3 PUFA supplements, vitamin D supplements, in addition to adhering to all instructions concerning the study procedures). Subjects were told to maintain their habitual physical activity level throughout the study. The following exclusion criteria were also adopted: any previously diagnosed gastrointestinal disease, type 1 or 2 diabetes mellitus, antibiotic or steroid medication within the last four months, intake of probiotics within the last four weeks, frequent use of non-steroidal anti-inflammatory drugs, intake of any other drugs affecting inflammation and the gastrointestinal barrier, rigorous physical exercise more than four hours/week, known allergy to fish, soy or milk, regular smoking, alcohol consumption of more than 108 g pure alcohol per week or more than 36 g pure alcohol per occasion, as well as pregnancy and breastfeeding. These inclusion and exclusion criteria remained unchanged throughout the study.

The study was conducted according to the Declaration of Helsinki at the Center for Clinical Nutrition Stuttgart in 2019 and 2020 and has been approved by the local Ethical Committee (Ethik-Kommission der Landesärztekammer Baden-Württemberg, F-2018-105), and was registered at Clinical-Trials.gov (NCT04126330).

At a baseline visit (W0) the participants received information about the study and signed an informed consent form, the BMI was calculated, and blood samples (approximately 4 ml) were obtained for hs-CRP measurement. If the participants fulfilled all inclusion (and no exclusion criteria) they were subsequently invited to attend a second visit (i.e. baseline / W0) where all baseline parameters were assessed. Before attending this visit, all included participants had picked up the instructions and material for the faecal sample collection and the urine recovery test, all of which were collected in their home before attending W0. Four weeks after W0 they came back for the third visit (W4) and after another four weeks (eight weeks after W0) they completed their final visit (W8). All study products were distributed at W0, but before the supplementation commenced, fasting blood samples were taken to analyse hs-CRP, other blood markers, and FA. Also, the faecal samples and urine collected at home (subsequently used for SCFA and the barrier permeability measurements) were handed in to the research team. Furthermore, the WOMAC and food frequency questionnaire (FFQ) were filled out and the sit stand test (SST) was completed. At W4, fasting blood samples were obtained for hs-CRP and other blood markers, and the participants received the instructions and material for the urine and faecal samples, to be handed in at the W8 visit. Apart from filling out the FFQ, the W8 visit was the same as the baseline visit; in addition, participants returned what was left of their study products (i.e. cans and pills), which were later used for evaluation of participant compliance.

**Table 1 shows the participant baseline characteristics:**

| | | Placebo (n = 39) | | | Treatment (n = 37) | | | p-value |
|---|---|---|---|---|---|---|---|---|
| Women/ men (n) | | 30/9 | | | 30/7 | | | |
| Anthropometry | | | | | | | | |
| Age [years] | | 49 | ± | 10 | 50 | ± | 11 | 0.695 |
| Weight [kg] | | 97.6 | ± | 12.3 | 100.9 | ± | 14 | 0.276 |
| BMI [kg/m2] | | 33.8 | ± | 3.6 | 35.0 | ± | 2.9 | 0.133 |
| | n overweight | 8 | | | 0 | | | |
| | n obese | 31 | | | 37 | | | |

| Blood biomarkers | | | | | | | | |
|---|---|---|---|---|---|---|---|---|
| Hs-CRP [mg/l] | | 4.1 | ± | 2.0 | 4.4 | ± | 2.1 | 0.433 |
| γ -GT [U/l] | | 33 | ± | 24 | 29 | ± | 15 | 0.578 |
| AST [U/l] | | 30 | ± | 31 | 26 | ± | 11 | 0.932 |
| ALT [U/l] | | 28 | ± | 17 | 29 | ± | 14 | 0.445 |
| Plasma glucose [mg/dl] | | 90 | ± | 10 | 95 | ± | 14 | 0.123 |
| Insulin [µE/ml] | | 19.0 | ± | 15.7 | 25.2 | ± | 28.5 | 0.856 |
| HOMA index | | 4.2 | ± | 3.5 | 6.2 | ± | 7.8 | 0.735 |
| Creatinine [mg/dl] | | 0.83 | ± | 0.15 | 0.79 | ± | 0.15 | 0.253 |
| Uric acid [mg/dl] | | 5.9 | ± | 1.4 | 5.6 | ± | 1.1 | 0.295 |
| Cholesterol [mg/dl] | | 236 | ± | 53 | 231 | ± | 42 | 0.660 |
| Triglycerides [mg/dl] | | 169 | ± | 78 | 148 | ± | 64 | 0.257 |
| HDL-cholesterol [mg/dl] | | 52 | ± | 12 | 53 | ± | 11 | 0.294 |
| LDL-cholesterol [mg/dl] | | 154 | ± | 39 | 150 | ± | 30 | 0.619 |
| LDL/HDL cholesterol quotient | | 3.1 | ± | 0.9 | 2.9 | ± | 0.7 | 0.367 |

| FFQ | | | | | | | | |
|---|---|---|---|---|---|---|---|---|
| Energy [kcal] | | 1904.0 | ± | 867.2 | 1816.0 | ± | 896.4 | 0.560 |
| Fat [g] | | 75.4 | ± | 41.6 | 75.3 | ± | 40.0 | 0.890 |
| Saturated fatty acids [g] | | 34.6 | ± | 20.4 | 36.0 | ± | 20.8 | 0.687 |
| Polyunsaturated fatty acids [g] | | 10.8 | ± | 5.8 | 9.5 | ± | 5.0 | 0.299 |
| Short chain fatty acids [g] | | 1.7 | ± | 1.2 | 1.9 | ± | 1.2 | 0.641 |
| Vitamin D [µg] | | 4.2 | ± | 3.2 | 3.8 | ± | 2.5 | 0.832 |

| | | | | | | | | |
|---|---|---|---|---|---|---|---|---|
| NB: Values are expressed as mean ± standard deviation (SD). Abbreviations: BMI, body mass index; hs-CRP, high-sensitivity C-reactive protein; γ-GT, gamma-glutamyl transferase; AST, aspartate aminotransferase; ALT, alanine transaminase; HOMA index, Homeostasis Model Assessment for Insulin Resistance; HDL, high-density lipoprotein; LDL, low-density lipoprotein; FFQ, Food Frequency Questionnaire. Statistics: Comparison of groups revealed no difference for all parameters listed in the table (p > 0.05, unpaired t-test). | | | | | | | | |

Concerning BMI, there were eight (20.5%) overweight (BMI 25-29.9 kg/m²) individuals in the placebo group and none in the treatment group, while thirty-one participants (79.5%) were obese (BMI ≥30 kg/m²) in the placebo group and 37 (100%) in the treatment group (Table 1); there was, however, no statistically significant difference in BMI between the two groups. Notably, no laboratory parameter was significantly different at baseline between the two groups. Furthermore, the FFQ showed no difference between the two groups.

For the evaluation of physical function, the sit stand test (SST) was conducted and the WOMAC questionnaire completed by participants at W0 and W8. WOMAC register self-reported signs of physical disability and relevant changes in health status; it was initially developed for patients with osteoarthritis, but may be used in other populations to evaluate the effects of interventions. The questionnaire consists of three scales: pain, stiffness, and function. The higher the WOMAC value, the higher the pain, stiffness, and functional limitations; the maximum score is 96 (maximum score for pain is 20, 8 for stiffness, and 68 for functional limitations).

The active study product consisted of 1100 mg fish oil 640 mg omega-3 FAs (300 mg EPA) and 220 mg DHA) (dosed as 2 capsules), and 200 IU of Vitamin D (VNP Active n-3 PUFA manufactured by Pfizer CH). The probiotic product contained 1.25 × 10⁹ colony-forming units (CFU) (dosed as 2 capsules, i.e. 10 billion CFUs in total) of each of *Lactobacillus paracasei* (LCP-37), *Lactobacillus acidophilus* (NCFM^{®}), *Bifidobacterium lactis* (Bi-07) and *Bifidobacterium lactis* (Bi-04) (Bifiform Daily Plus, supplied by Pfizer CH, manufactured by Danisco, DuPont). The control group received maltodextrin capsules for the probiotic placebo and capsules with sunflower oil for the omega-3 placebo. During the eight-week intervention, the participants had to consume four capsules daily before breakfast at the same time each day. The placebo products had the same packaging as the study product and were manufactured on behalf of Pfizer Consumer Health Care (same as the active study products). To meet the compliance criteria, participants had to achieve a compliance of 85% and should have taken the supplements according to the instructions at least five days (out of seven) before the study visit which was documented by a daily dairy.

Randomization and blinding were performed by team members not involved in the study. Thus, both participants and any study personnel who were in contact with participants were blinded. Blinding was maintained until the statistical analyses were completed. The randomization was generated using the Randlist software (datinf GmbH, Tübingen, Germany, available at randomisa-tion.eu) and performed in blocks of four. Randomization numbers were sequentially noted on the study product containers. The study personnel handed over the lowest number available to females eligible for the study, and the highest number available to males eligible for the study. In this manner, an equal distribution of both sexes into the two arms was ensured.

**Table 2:**

| | **Placebo** | | | **Treatment group** | | | **Between groups** | |
|---|---|---|---|---|---|---|---|---|
| | **W0** | **W8** | **A W8-W0 Treatment effect** | **W0** | **W8** | **Δ W8-W0 Treatment effect** | **p-value W8** | **p-value Δ W8-W0 Treatment effect** |
| **Inflammatory markers** | | | | | | | | |
| hs-CRP [mg/l] | 4.4 ± 3.9 | 4.9 ± 3.5 | 0.51 ± 4.84 | 4.2 ± 2.4 | 5.5* ± 3.8 | 1.34 ± 3.31 | 0.394 | 0.159 |
| IL-6 [pg/ml] | 0.8 ± 0.5 | 0.9 ± 0.6 | 0.08 ± 0.37 | 1.0 ± 0.9 | 0.9* ± 0.8 | -0.04 ± 0.62 | 0.477 | 0.848 |
| TNF-α [pg/ml] | 1.4 ± 0.7 | 1.4 ± 0.8 | 0.08 ± 0.33 | 1.3 ± 0.6 | 1.4 ± 0.7 | 0.06 ± 0.27 | 0.666 | 0.326 |
| IFN-γ [pg/ml] | 5.0 ± 2.7 | 6.3 ± 5.3 | 1.33 ± 5.44 | 5.3 ± 4.4 | 5.4 ± 4.1 | 0.10 ± 2.85 | 0.287 | 0.26 |
| IL-4 [pg/ml] | 0.0 ± 0.0 | 0.0 ± 0.0 | 0.00 ± 0.01 | 0.0 ± 0.0 | 0.0 ± 0.0 | 0.00 ± 0.02 | 0.477 | 0.427 |
| IL-8 [pg/ml] | 11.4 ± 6.3 | 11.5 ± 5.7 | 0.03 ± 2.48 | 19.9 ± 43.8 | 19.6 ± 39.2 | -0.38 ± 5.46 | 0.856 | 0.856 |
| IL-12 [pg/ml] | 0.1 ± 0.1 | 0.1 ± 0.2 | 0.02 ± 0.16 | 0.2 ± 0.6 | 0.2 ± 0.6 | 0.02 ± 0.09 | 0.249 | 0.86 |
| IL-10 [pg/ml] | 0.3 ± 0.4 | 0.3 ± 0.3 | 0.03 ± 0.22 | 0.2 ± 0.1 | 0.2 ± 0.1 | 0.0 ± 0.07 | 0.131 | 0.629 |

| **Laboratory parameters** | | | | | | | | |
|---|---|---|---|---|---|---|---|---|
| weight [kg] | 97.2 ± 12.1 | 97.4 ± 12.1 | 0.15 ± 2.02 | 101.1 ± 14.3 | 100.5 ± 14.2 | 0.17 ± 2.28 | 0.316 | 0.766 |
| BMI [kg/m2] | 33.7 ± 3.5 | 33.8 ± 3.5 | 0.06 ± 0.7 | 35.0 ± 2.8 | 35.1 ± 2.7 | 0.05 ± 0.78 | 0.083 | 0.512 |
| vitamin D [ng/ml] | 22.4 ± 11.8 | 22.1 ± 10.6 | -0.33 ± 5.85 | 24.1 ± 15.7 | 24.6 ± 10.9 | 0.51 ± 8.05 | 0.259 | 0.359 |
| glucose [mg/dl] | 91 ± 10 | 93* ± 12 | 1.97 ± 6.27 | 94 ± 12 | 94 ± 13 | 0.32 ± 6.28 | 0.505 | 0.256 |
| insulin [µE/ml] | 14.8 ± 7.5 | 14.9 ± 7.3 | 0.04 ± 5.14 | 15.2 ± 8.5 | 14.7 ± 7.7 | -0.4 ± 5.07 | 0.687 | 0.664 |
| HOMA index | 3.3 ± 1.8 | 3.4 ± 1.8 | 0.06 ± 1.35 | 3.5 ± 2.1 | 3.5 ± 1.9 | 0.02 ± 1.44 | 0.903 | 0.738 |

| | | | | | | | | |
|---|---|---|---|---|---|---|---|---|
| Statistics: * differences between week 0 and W8 within the placebo group and the treatment and p-value at W8 as group difference. *p < 0.05. | | | | | | | | |

Effect of Supplementation on hs-CRP Levels and other inflammatory markers: Considering hs-CRP, there was an increase from 4.2 ± 2.4 mg/L at W0 to 5.5 ± 3.8 mg/L at W8 in the treatment group (p = 0.018) and no changes in the placebo group (Figure 2A). Nevertheless, when comparing the two groups after treatment, there is no significant difference in mean hs-CRP levels nor in treatment effects. The IL-6 level decreased within the treatment group from 1.0 ± 0.9 pg/mL at W0 to 0.9 ± 0.8 pg/mL at W8 (p = 0.034), however, no difference between groups nor a significant treatment effect was measured (Figure 2B).

No other parameters of inflammation, such as TNF-o, IFN-γ, and IL-4, -8, or -12, differed between the groups or were changed post-intervention (Table 2). No change was observed between the groups for BMI, insulin or the HOMA index (Table 2). The glucose level increased within the placebo group (p = 0.041), while no changes were found within the treatment group (p = 0.650), nor between groups. Regarding the vitamin D levels, no significant change between groups was found, however, there was a modest increase of Δ0.51 ± 8.05 ng/mL in the treatment group and a slight decrease in the placebo group (Δ -0.33 ± 5.85 ng/mL) over the study period.

Regarding the ratio of inflammatory markers n-6 to n-3, a significant reduction (i.e. lower n-6 as compared to n-3 levels) was observed in the treatment group, as compared to the placebo group (Figure 2C). The AA:EPA ratio was also reduced within the treatment group, from 9.3 ± 5.3 at W0 to 4.5±3.0 at W8 (p < 0.001, Figure 2D). The ratio after the intervention at W8 was lower in the treatment group compared to the placebo group (p < 0.001). Regarding the total n-3 FAs, the treatment group showed higher values at W0 and W8 compared to the placebo group, as well as a significantly higher treatment effect compared to the placebo group. Also, the plasma n-6 FA level increased in both groups during the study (treatment group: p < 0.001; placebo group: p < 0.01) but no difference was measured between the treatment effect between both groups.

**Physical function tests, Table 3:**

| | **Placebo** | | **Treatment** | | | | **Between groups** | | | |
|---|---|---|---|---|---|---|---|---|---|---|
| | **W0** | **W8** | **Δ W8-W0 Treatment effect** | | **W0** | **W8** | **Δ W8-W0 Treatment effect** | | **W8** | **A W8-W0 Treatment effect** |
| **Physical function tests** | | | | | | | | | | |
| 5 STS [s] | 10.9 ± 3.2 | 10.1* ± 3.3 | -0.75 | ± 1.78 | 11 ± 3.1 | 10.3* ± 2.9 | -0.67 | ± 2.38 | 0.503 | 0.823 |
| WOMAC score | 14.1 ± 14.5 | 13.3 ± 16.7 | -0.8 | ± 6.8 | 19.1 ± 17.7 | 15.8* ± 17.7 | -3.4 | ± 7.1 | 0.346 | 0.095 |

The SST time decreased in both groups, from 11.0 ± 3.1 s to 10.3 ± 2.9 s (p = 0.041) in the treatment group and from 10.9 ± 3.2 s to 10.1 ± 3.3 s (p < 0.001) in the placebo group; no difference between the two groups were found (Figure 2E).

However, the WOMAC score decreased from 19.1 ± 17.7 to 15.8 to 7.7 in the treatment group (p = 0.006) and showed no changes in the placebo group. There was, however, no significant difference between the groups (Figure 2F; Table 3).

The present study aimed to investigate the effect of an eight-week daily supplementation with probiotics, EPA+DHA, and vitamin D on low-grade inflammation in overweight and obese individuals. The study showed no difference in hs-CRP levels. However, IL-6 levels decreased in the treated subject's post-intervention reduction which may indicate some modest effects on inflammation. In addition, the intervention seemed to elicit some improvements in mobility, as evaluated by self-reported WOMAC scores, in the treatment group.

Obesity is usually associated with reduced mobility. Therefore, an improvement in mobility markers is beneficial. The study showed an improvement in the WOMAC score in the treatment group. This improvement could possibly indicate a subtle anti-inflammatory effect leading to a reduction in pain during certain movements. Results from other studies have shown similar effects after supplementation with n-3 PUFAs [1], probiotics [2], and vitamin D [3]. Our subgroup correlation analysis showed a positive association between age and WOMAC score, which worsens with age, as expected. This association may suggest that the pain-reducing effect is more important in older people.

However, many overweight people of all ages have limited mobility. The results of the SST suggest an improvement in mobility after the intervention in both groups, which could be due to a 'learning effect'. Overall, further mobility markers should be investigated in future studies. Although the results have modest effects, an improvement in mobility markers in this population is a particularly valuable effect. An anti-inflammatory effect may be associated with more exercise and therefore possible weight loss, leading to less stress on the knees and hips. Without wishing to be bound by theory, supplementation may reduce pain and inflammation, which in turn leads to better mobility.
[Reference 1]. Hill, C.L.; March, L.M.; Aitken, D.; Lester, S.E.; Battersby, R.; Hynes, K.; Fedorova, T.; Proudman, S.M.; James, M.; Cleland, L.G.; et al. Fish Oil in Knee Osteoarthritis: A Randomised Clinical Trial of Low Dose versus High Dose. Ann Rheum Dis 2016, 75, 23-29, doi:10.1136/annrheumdis-2014-207169.
[Reference 2]. Lei, M.; Guo, C.; Wang, D.; Zhang, C.; Hua, L. The Effect of Probiotic Lactobacillus Casei Shirota on Knee Osteoarthritis: A Randomised Double-Blind, Placebo-Controlled Clinical Trial. Beneficial Microbes 2017, 8, 697-703, doi:10.3920/BM2016.0207.
[Reference 3]. Gao, X.-R.; Chen, Y.-S.; Deng, W. The Effect of Vitamin D Supplementation on Knee Osteoarthritis: A Meta-Analysis of Randomized Controlled Trials. International Journal of Surgery 2017, 46, 14-20, doi:10.1016/j.ijsu.2017.08.010.

## Claims

1. A combination of (i) *Bifidobacterium animalis, Bifidobacterium lactis, Lactobacillus paracasei* and *Lactobacillus acidophilus;* (ii) one or more Omega-3 fatty acids; and (iii) Vitamin D for use in improving mobility and physical function in a human subject by oral administration.

2. The combination for use according to Claim, wherein the one or more Omega-3 fatty acids comprise eicosapentaenoic acid (EPA) and docosahexaenoic acid (DHA).

3. The combination for use according to Claim 1 or 2, wherein the Vitamin D is vitamin D3 (cholecalciferol) and/or vitamin D2 (ergocalciferol), preferably vitamin D3.

4. The combination for use according to Claim 3, wherein the combination comprises at least 2.5×10⁹ colony forming units (CFU) of each of *Bifidobacterium animalis, Bifidobacterium lactis, Lactobacillus paracasei* and *Lactobacillus acidophilus.*

5. The combination for use according to Claim 4, wherein the combination comprises at least 300mg of EPA and at least 220mg of DHA.

6. The combination for use according to Claim 5, wherein the one or more Omega-3 fatty acids are obtained from fish oil.

7. The combination for use according to Claim 5 or 6, wherein the combination comprises at least 200 IU of Vitamin D.

8. The combination for use according to any preceding claim, wherein the human subject has a body mass index (BMI) of at least 25 kg/m², particularly at least 28 kg/m².

9. The combination for use according to Claim 8, wherein the human subject has a body mass index (BMI) of from 28 kg/m² to 29.9 kg/m².

10. The combination for use according to Claim 8, wherein the human subject has a body mass index (BMI) of 30 kg/m² or more.

11. The combination for use according to Claim 10, wherein the human subject has a body mass index (BMI) of from 30 kg/m² to 40 kg/m².

12. The combination for use according to any preceding claim, wherein the human subject is at least 25 years of age.

13. The combination for use according to Claim 12, wherein the human subject is from 25 to 65 years of age.

14. The combination for use according to Claim 12 or 13, wherein the combination is a composition comprising 2.5×10⁹ colony forming units of *Bifidobacterium animalis,* 2.5×10⁹ colony forming units of *Bifidobacterium lactis,* 2.5×10⁹ colony forming units of *Lactobacillus paracasei,* 2.5×10⁹ colony forming units of *Lactobacillus acidophilus,* 300mg of EPA, 220mg of DHA and 200 IU of Vitamin D.

15. The combination for use according to Claim 12 or 13, wherein the combination is a kit comprising (i) a probiotic composition comprising 2.5×10⁹ colony forming units of *Bifidobacterium animalis,* 2.5×10⁹ colony forming units of *Bifidobacterium lactis,* 2.5×10⁹ colony forming units of *Lactobacillus paracasei,* 2.5×10⁹ colony forming units of *Lactobacillus acidophilus* and (ii) a fish oil composition comprising 300mg of EPA, 220mg of DHA and 200 IU of Vitamin D.

16. The combination for use according to Claim 15, wherein the probiotic composition is in the form of a capsule, a gel, a liquid dispersion, a pill, a powder, or a suspension.

17. The combination for use according to Claim 15 or 16, wherein the fish-oil composition is in the form of a capsule, a gel, a liquid dispersion, a pill, a powder, or a suspension.

18. The combination for use according to Claim 15, 16 or 17 wherein the probiotic composition is administered in two doses per day, each dose comprising 1.25×10⁹ colony forming units of *Bifidobacterium animalis,* 1.25×10⁹ colony forming units of *Bifidobacterium lactis,* 1.25×10⁹ colony forming units of *Lactobacillus paracasei* and 1.25×10⁹ colony forming units of *Lactobacillus acidophilus.*

19. The combination for use according to Claim 15, 16, 17 or 18, wherein the fish-oil composition is administered in two doses per day, each dose comprising 150mg of EPA, 110mg of DHA and 100 IU of Vitamin D.
